(19)

Europäisches
Patentamt
European
Patent Office
Office européen
des brevets

(11)  **EP 3 351 301 A1**

(12)  **EUROPEAN PATENT APPLICATION**
published in accordance with Art. 153(4) EPC

(43) Date of publication:
**25.07.2018  Bulletin 2018/30**

(21) Application number: **16846581.3**

(22) Date of filing: **15.09.2016**

(51) Int Cl.:
*B01J 23/887* [(2006.01)]    *B01J 37/02* [(2006.01)]
*B01J 37/08* [(2006.01)]

(86) International application number:
**PCT/JP2016/077314**

(87) International publication number:
**WO 2017/047710 (23.03.2017 Gazette 2017/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **16.09.2015  JP 2015183438**

(71) Applicant: **Nippon Kayaku Kabushiki Kaisha
Tokyo 100-0005 (JP)**

(72) Inventors:
• **OKUMURA Shigeki
Sanyoonoda-shi
Yamaguchi 757-0002 (JP)**

• **NAKAZAWA Yuta
Sanyoonoda-shi
Yamaguchi 757-0002 (JP)**
• **MOTOMURA Hiroki
Sanyoonoda-shi
Yamaguchi 757-0002 (JP)**
• **NISHIZAWA Bungo
Sanyoonoda-shi
Yamaguchi 757-0002 (JP)**
• **OBATA Tomohiro
Sanyoonoda-shi
Yamaguchi 757-0002 (JP)**
• **NAKAYAMA Koji
Sanyoonoda-shi
Yamaguchi 757-0002 (JP)**

(74) Representative: **Gille Hrabal
Brucknerstrasse 20
40593 Düsseldorf (DE)**

(54)  **CATALYST FOR PRODUCTION OF CONJUGATED DIOLEFIN AND METHOD FOR PRODUCING SAME**

(57)  A supported molded catalyst having increased hardness, the supported molded catalyst being capable of improving the long-term stability of a reaction for producing a conjugated diolefin by catalytic oxidative dehydrogenation from a mixed gas including a monoolefin having 4 or more carbon atoms and molecular oxygen; and a method for producing the catalyst is provided. A molded catalyst for conjugated diolefin production, the molded catalyst being a catalyst for producing a conjugated diolefin by a catalytic oxidative dehydrogenation reaction from a mixed gas including a monoolefin having 4 or more carbon atoms and molecular oxygen, and being produced by molding a composite metal oxide and a glass fiber-like inorganic auxiliary agent.

**EP 3 351 301 A1**

**Description**

Technical Field

The present invention relates to a catalyst with which a conjugated diolefin is produced by a catalytic oxidative dehydrogenation reaction from a mixed gas including a monoolefin having 4 or more carbon atoms and molecular oxygen, and a method for producing the catalyst.

Background Art

Butadiene, which is a raw material for a synthetic rubber or the like, is conventionally industrially produced by thermal cracking and extraction of a naphtha fraction; however, since there is a concern that stable supply to the market in the future may be aggravated, there is a demand for new methods for producing butadiene. Thus, attention has been paid to a method of oxidatively dehydrogenating n-butene from a mixed gas including n-butene and molecular oxygen in the presence of a catalyst. However, since a coke-like material based on the intended product and/or reaction byproducts is precipitated on or attached to the catalyst surface or an inert material, the interior of the reaction tube, or the interior of the facility for a subsequent process, it is concerned that various problems may occur, such as inhibition of the flow of a reaction gas in an industrial plant, blocking of the reaction tube, and shoot-down of the plant or a decrease in the yield resulting from such inhibition or blocking. For the purpose of avoiding the above-mentioned problems, in an industrial plant, a regeneration treatment by which the reaction is stopped before blocking occurs, and the coke-like material is removed by combustion by means of the temperature increase at the blocked site in the reaction tube, the facility of the subsequent process or the like, is generally performed. However, since stopping a reaction that is under steady operation and performing a regeneration treatment leads to deterioration of the economic efficiency in an industrial plant, it is desired to suppress the occurrence of the coke-like material as much as possible.

Furthermore, another problem to be solved in regard to the method for producing butadiene may be damage of the catalyst. This is a phenomenon characteristic to a process for producing a conjugated diolefin, in which the shape of the catalyst is changed or deteriorated from the shape of the catalyst at the time of packing to catalyst pieces, namely, a flaky form, a granular form, or even a powdered form, due to a long-term reaction and the catalyst disintegrates (damaged), and the problems that will be mentioned below are concerned. That is, there are concerns about an increase in the pressure loss caused by damaged catalyst pieces accumulating inside the reactor, an undesirable side reaction caused by catalyst pieces that have locally accumulated inside the reactor, incorporation of damaged catalyst pieces into the purification system in the subsequent stages, and the like, and the measures taken in the conventional technologies are known as in the literatures disclosed below.

Patent Literature 1 discloses a correlation between the change ratio of the outer diameters of the catalyst measured before and after a reaction and the change in strength. Tablet molding, which is the molding method used for the catalyst of Patent Literature 1, allows the catalyst to be molded so as to have high mechanical strength of catalyst and to have the catalytically active components densely aggregated. Therefore, tablet molding has problems such as that a coke-like material is likely to be produced by side reactions and/or retained in the interior of the catalyst, and the coke-like material is easily precipitated out compared to catalysts produced by other molding methods; that the heat of reaction is likely to be accumulated in the interior of the catalyst, and a decrease in yield or a runaway reaction occurs therein; and that the productivity for the catalyst itself is poor. Patent Literature 2 discloses a correlation between the crush rate in the packed catalyst and the amount of production of a coke-like material; however, there is no disclosure on the catalyst or reaction conditions for suppressing the crush rate in a long-term reaction.

As a means for solving the problem of damage to the catalyst, increase of the hardness of the catalyst itself may be considered. The degree of attrition resistance that will be described below may be mentioned as an evaluation method for mechanical strength that has been hitherto used by those ordinarily skilled in the art; however, this is a physical value representing the extent of damage against impact at the time of catalyst packing, and the problems described below may be considered as to the appropriateness of the method for the evaluation of mechanical strength. That is, in an evaluation of the degree of attrition resistance, since the mechanical load exerted on the catalyst is low, damage caused by a long-term reaction has been observed even in catalysts with satisfactory degrees of attrition resistance, as will be described below. In contrast, in an evaluation of hardness using a tensile compression testing machine, since the correlation between the hardness and the damage of the catalyst caused by a long-term reaction may be significantly confirmed as will be described below, a physical property evaluation that is more difficult and more suitable than the evaluation of the degree of attrition resistance, which is a conventional evaluation of mechanical strength, may be carried out. Furthermore, as will be described below, even in the case of a catalyst having a satisfactory degree of attrition resistance, it may be seen that the hardness is low, and the catalyst is damaged by a long-term reaction. Thus, a method for physical property evaluation representing a correlation with the damage of the catalyst caused by a long-term reaction was not known.

Regarding general methods for increasing the mechanical strength in connection with a molded catalyst, the following literatures are known.

Patent Literature 3 to Patent Literature 9 all relate to catalysts obtained by adding an organic auxiliary agent or/and an inorganic auxiliary agent having a particular particle size distribution, a particular fiber length, a particular acid strength, or the like, and methods for producing the catalysts. In these patent literatures, the method for evaluating the mechanical strength corresponds to an evaluation of the powdering ratio caused by packing or the degree of attrition resistance, and the effect on the increase in hardness is not clearly known. That is, in regard to a molded catalyst, particularly a supported molded catalyst, any findings about the type or the amount of addition of the auxiliary agent that particularly increases hardness among the mechanical strength characteristics are not described in Patent Literature 3 to Patent Literature 9, and the findings are not known even to those ordinarily skilled in the art.

When a catalyst is perceived as a ceramic material, control of the pore structure as in the case of Non-Patent Literature 1 may be considered as the method for increasing hardness in addition to the addition of an inorganic auxiliary agent. It is thought that damage of a catalyst is attributed to the stress inside the catalyst during a long-term reaction or a regeneration treatment, and it is speculated that stress increases in the vicinity of pores, finally causing damage. That is, in order to suppress damage of the catalyst, it is preferable to reduce pores; however, Non-Patent Literature 1 does not have any description that hardness is increased without adding an organic auxiliary agent to a supported molded catalyst, and thus, this is not publicly known.

From the viewpoint of economic efficiency in industrial plants, it is also definitely important that the intended product, butadiene, is obtained with high yield. When the butadiene yield is low, it is implied that the yield of byproducts is relatively high; however, in this case, in order to obtain butadiene of high purity as the final manufactured product in an industrial plant, a purification system having superior performance is required, and it is concerned that high facility cost of the purification system may be needed. It is concerned that undesirable side reactions may occur as a result of addition of an auxiliary agent for increasing hardness. That is, there is a demand for the development of a catalyst which is not accompanied by a decrease in the amount of production of butadiene or an increase in the amount of production of byproducts, and with which the damage of the catalyst caused by a long-term reaction is suppressed.

Citation List

Patent Literature

Patent Literature 1: JP 2011-241208 A
Patent Literature 2: JP 2012-046509 A
Patent Literature 3: JP 3313863 B
Patent Literature 4: JP 4863436 B
Patent Literature 5: JP 2002-273229 A
Patent Literature 6: JP 5388897 B
Patent Literature 7: WO 2012/036038 A
Patent Literature 8: JP 5628936 B
Patent Literature 9: JP 07-16463 A

Non Patent Literature

Non Patent Literature 1: MIYATA, Noboru and KANNO, Hiroshi, "Zairyo (Materials)", 32, 354, p.102-108

Summary of Invention

Technical Problem

An object of the present invention is to provide a catalyst having increased hardness, which may improve the long-term stability of a reaction in a reaction for producing a conjugated diolefin by catalytic oxidative dehydrogenation from a mixed gas including a monoolefin having 4 or more carbon atoms and molecular oxygen, and to provide a method for producing the catalyst.

Solution to Problem

The present inventors conducted a thorough investigation in order to solve the problems described above, and as a result, the inventors found that when a glass fiber-like inorganic auxiliary agent is added, hardness may be increased without lowering the yield of a conjugated diolefin, which is an intended product, and damage of the catalyst caused by a long-term reaction may be noticeably suppressed. Thus, the inventors found that the problems described above may be solved, and thus completed the present invention.

The present invention has the following features from (1) to (7) singly or in combination. That is, the present invention relates to:

(1) a molded catalyst for conjugated diolefin production, the catalyst being used for producing a conjugated diolefin by a catalytic oxidative dehydrogenation reaction from a mixed gas including a monoolefin having 4 or more carbon atoms and molecular oxygen, and being obtained by molding a composite metal oxide and a glass fiber-like inorganic auxiliary agent;

(2) the molded catalyst for conjugated diolefin production according to (1), wherein the requirement of the following Formula (A) is satisfied:

$$R \ (= La/Dc) \ \leq \ 45 \qquad\qquad (A)$$

wherein La represents the average fiber length of the glass fiber-like inorganic auxiliary agent; and Dc represents the average particle size of the composite metal oxide;

(3) the molded catalyst for conjugated diolefin production according to (1) or (2), wherein the molded catalyst does not include an organic auxiliary agent;

(4) the molded catalyst for conjugated diolefin production according to any one of (1) to (3), wherein the composite metal oxide satisfies the following Compositional Formula (D) :

$$Mo_{12}Bi_aFe_bCo_cNi_dX_eY_fZ_g \qquad\qquad (D)$$

wherein X represents at least one element of alkali metals selected from lithium, sodium, potassium, rubidium, and cesium; Y represents at least one element of alkaline earth metals selected from magnesium, calcium, strontium, and barium; Z represents at least one element selected from lanthanum, cerium, praseodymium, neodymium, samarium, europium, antimony, tungsten, lead, zinc, cerium, and thallium; a, b, c, d, e, and f represent the atomic ratios of bismuth, iron, cobalt, nickel, X, Y, and Z, respectively, with respect to molybdenum 12; and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$, g represents a value satisfying the oxidation state of the other elements;

(5) the supported molded catalyst for conjugated diolefin production according to any one of (1) to (4), wherein the supported molded catalyst is a catalyst for producing a conjugated diolefin by a catalytic oxidative dehydrogenation reaction from a mixed gas including a monoolefin having 4 or more carbon atoms and molecular oxygen, and is produced by supporting a composite metal oxide and a glass fiber-like inorganic auxiliary agent on a carrier;

(6) a method for producing the molded catalyst for conjugated diolefin production according to any one of (1) to (5), the method including steps of preparing a mixed solution or slurry including the compounds containing the various metals of the composite metal oxide under the conditions of a temperature of from 20°C to 90°C; subsequently drying and preliminarily calcining the mixture; molding the mixture together with a glass fiber-like inorganic auxiliary agent; and subjecting the molded product to main calcination;

(7) the method for producing the molded catalyst for conjugated diolefin production according to (6), wherein the temperature of preliminary calcination is from 200°C to 600°C, and the main calcination temperature is from 200°C to 600°C;

(8) the method for producing the supported molded catalyst for conjugated diolefin production according to (6) or (7), the method including a molding step of coating a carrier with the composite metal oxide and the glass fiber-like inorganic auxiliary agent together with a binder, wherein the support ratio of the catalytically active components is from 20% by weight to 80% by weight, and the average particle size of the catalyst is from 3.0 mm to 10.0 mm; and

(9) the method for producing the molded catalyst according to any one of (6) to (8), wherein an organic auxiliary agent is not used in the entire production process.

Advantageous Effects of Invention

According to the present invention, a catalyst having high hardness, which may be used in a reaction for producing a conjugated diolefin by catalytic oxidative dehydrogenation from a mixed gas including a monoolefin having 4 or more carbon atoms and molecular oxygen, is obtained, and since damage of the catalyst during a long-term reaction is suppressed, a catalyst having long-term stability and a method for producing the same may be provided.

Description of Embodiments

A catalyst that may be used in a reaction for producing a conjugated diolefin by a catalytic oxidative dehydrogenation reaction from a mixed gas including a monoolefin having 4 or more carbon atoms and molecular oxygen, and preferably may be used in a reaction for producing butadiene by a catalytic oxidative dehydrogenation reaction from a mixed gas including n-butene and molecular oxygen, and a method for producing the catalyst will be described, and hereinafter, a catalyst for producing a conjugated diolefin of the present invention, the catalyst being molded by adding a glass fiber-like inorganic auxiliary agent, and a method for producing the catalyst will be described.

According to the present invention, n-butene means a gas of a single component selected from among 1-butene, trans-2-butene, cis-2-butene, and isobutylene, or a mixed gas including at least one of the components, and more strictly, the term butadiene means 1,3-butadiene.

The glass fiber-like inorganic auxiliary agent used for the present catalyst is an arbitrary fiber-like or rod-like auxiliary agent produced mainly by subjecting any arbitrary naturally-occurring and/or artificial inorganic substance that is not destroyed by fire even by a heat treatment at 600°C, to a treatment at a temperature higher than or equal to the glass transition temperature, and it should be noted that not the entirety of the agent is destroyed by fire during the main calcination process that will be described below. Since a glass fiber-like inorganic auxiliary agent remains even in the main calcination process that will be described, the agent plays a role of binding composite metal oxides (preliminarily calcined powder), and thus, there is obtained an effect of suppressing damage even when the load exerted upon damage is produced in the catalyst. According to the present invention, regarding the material for the glass fiber-like inorganic auxiliary agent, the Mohs hardness is not particularly limited; however, for example, among those products obtained by subjecting any one of arbitrary sulfide minerals, oxide minerals, halide minerals, inorganic acid salt minerals and organic minerals, or a combination thereof, to a heat treatment at a temperature higher than or equal to the glass transition temperature, any product having a Mohs hardness of 2 or higher (present glass) is preferred, and regarding the raw material of these materials, inorganic acid salt minerals are more preferred, while a silicate mineral is most preferred, examples of which may include, but are not limited to, glass fibers or chopped strands. Furthermore, when the glass fiber-like inorganic auxiliary agent is subjected to an acid treatment, an alkali treatment, a silane treatment and the like singly or in combination, the glass fiber-like inorganic auxiliary agent becomes suitable from the viewpoint of becoming inert to a catalytic reaction.

Furthermore, it is preferable that the average fiber length of the glass fiber-like inorganic auxiliary agent satisfies the conditions represented by the following Formula (A), it is more preferable that the average fiber length satisfies the conditions represented by the following Formula (B), and it is most preferable that the average fiber length satisfies the conditions represented by the following Formula (C). The glass fiber-like inorganic auxiliary agent is easily available from, for example, Central Glass Co., Ltd. or Nitto Boseki Co., Ltd. Also, the average particle size of the glass fiber-like inorganic auxiliary agent is preferably from 0.1 $\mu$m to 100 $\mu$m, and more preferably from 1 $\mu$m to 50 $\mu$m. The average fiber length of the glass fiber-like inorganic auxiliary agent is preferably from 10 $\mu$m to 4,000 $\mu$m, and more preferably from 50 $\mu$m to 500 $\mu$m.

$$R \ (= La/Dc) \leq 45 \qquad\qquad (A)$$

$$0.5 \leq R \ (= La/Dc) \leq 20 \qquad\qquad (B)$$

$$1 \leq R \ (= La/Dc) \leq 10 \qquad\qquad (C)$$

wherein La represents the average fiber length of the glass fiber-like inorganic auxiliary agent; and Dc represents the average particle size of the composite metal oxide).

According to the present invention, the average particle size of the composite metal oxide or the organic auxiliary agent is calculated by, for example, the following method.

There are no particular limitations on the apparatus; however, for example, LMS-2000e manufactured by Nishiyama Seisakusho Co., Ltd. is used. Various samples are introduced into cells using purified water as a dispersing medium, measurement is made at a scattered light intensity of about 4.0 to 6.0, and the average particle size is calculated from a particle size distribution obtained by the percentage by mass.

The average particle size of the composite metal oxide is preferably from 1 $\mu$m to 500 $\mu$m, and more preferably from 10 $\mu$m to 100 $\mu$m.

In regard to the present catalyst or the method for producing the same, it is preferable that an organic auxiliary agent is not added or used.

It is preferable that the composite metal oxide according to the present invention satisfies the following Compositional Formula (D):

$$Mo_{12}Bi_aFe_bCo_cNi_dX_eY_fZ_g \qquad (D)$$

wherein X represents at least one element of alkali metals selected from lithium, sodium, potassium, rubidium, and cesium; Y represents at least one element of alkaline earth metals selected from magnesium, calcium, strontium, and barium; Z represents at least one element selected from lanthanum, cerium, praseodymium, neodymium, samarium, europium, antimony, tungsten, lead, zinc, cerium, and thallium; a, b, c, d, e, and f represent the atomic ratios of bismuth, iron, cobalt, nickel, X, Y cerium, and Z, respectively, with respect to molybdenum 12; and in the ranges of $0.3 < a < 3.5$, $0.6 < b < 3.4$, $5 < c < 8$, $0 < d < 3$, $0 < e < 0.5$, $0 \le f \le 4.0$, and $0 \le g \le 2.0$, g represents a value that satisfies the oxidation states of the other elements.

There are no particular limitations on the raw materials of various metal elements for obtaining the present catalyst; however, nitrates, nitrites, sulfates, ammonium salts, organic acid salts, acetates, carbonates, subcarbonates, chlorides, inorganic acids, inorganic acid salts, heteropolyacids, heteropolyacid salts, hydroxides, oxides, metals, alloys, and the like containing at least one of various metal elements, or mixtures thereof may be used. Among these, preferred are nitrate raw materials.

There are no particular limitations on the method for producing the present catalyst; however, preferred is a method of obtaining a composite metal oxide, which is an active component of the catalyst, as a powder, and then molding the powder without adding or using an organic auxiliary agent, and the details will be described below. In the following description, a procedure of various processes will be described as a preferred example; however, there will be no limitations on the order of various steps for obtaining the final catalyst product, the number of steps, and the combination of various steps.

Step (A1) Compounding and drying

A mixed solution or a slurry of the raw materials of catalytically active components is prepared, the mixed solution or slurry is subjected to a process such as a precipitation method, a gelling method, a co-precipitation method, or a hydrothermal synthesis method, and then a dried powder of the present invention is obtained using a known drying method such as a dry spraying method, an evaporation solid-drying method, a drum drying method, or a freeze-drying method. This mixed solution or slurry may use any of water, an organic solvent, and a mixed solution thereof as the solvent, and the raw material concentrations of the active components of the catalyst are also not limited. Furthermore, there are no particular limitations on the compounding conditions and drying conditions, such as the liquid temperature of this mixed solution or slurry and the atmosphere; however, it is particularly preferable to select the conditions in appropriate ranges in consideration of the performance, mechanical strength and moldability of the final catalyst, the production efficiency, and the like. Among these, according to the present invention, the most preferred is a method of forming a mixed solution or a slurry of the raw materials of the active components of catalyst under the conditions of from 20°C to 90°C, introducing this into a spray drying machine, and regulating the hot air inlet temperature, the pressure inside the spray drying machine, and the flow rate of the slurry such that the drying machine outlet temperature is from 70°C to 150°C, and the average particle size of the dried powder thus obtainable is from 10 $\mu$m to 700 $\mu$m. Furthermore, in the processes from the compounding of the mixed solution or slurry of the present step to the drying, it is construed that adding the glass fiber-like inorganic auxiliary agent or/and organic auxiliary agent that will be described below in an arbitrary amount is also included in the present method for producing a catalyst.

Step (A2) Preliminary calcination

The dry powder thus obtained is preliminarily calcined at a temperature of from 200°C to 600°C, and thus the present composite metal oxide (preliminarily calcined powder) having an average particle size of from 10 $\mu$m to 100 $\mu$m may be obtained. According to the present invention, a composite metal oxide may be referred to as a preliminarily calcined powder. Also in regard to the conditions for this preliminary calcination, there are no particular limitations on the calcination time or the atmosphere at the time of calcination, and the technique for calcination is also not particularly limited and may be a fluidized bed, a rotary kiln, a muffle furnace, a tunnel calcination furnace, or the like. It is particularly preferable that these are selected in appropriate ranges in consideration of the performance, mechanical strength, and moldability of the final catalyst, the production efficiency, or the like. Among these, the most preferred for the present invention is a method of using a tunnel calcination furnace at a temperature in the range of from 300°C to 600°C for 1 hour to 12 hours in an air atmosphere. Furthermore, it is construed that adding the glass fiber-like inorganic auxiliary agent and the organic auxiliary agent that will be described below to the dry powder in arbitrary amounts before preliminary calcination or after preliminary calcination of the present step, is also included in the method for producing the present catalyst.

Step (A3) Molding

According to the present invention, the preliminarily calcined powder thus obtained is used after molding the powder. The shape of the molded article is not particularly limited and may be a spherical shape, a cylindrical shape, an annular shape, or the like; however, the shape must be selected in consideration of the mechanical strength of the catalyst that is finally obtained in a series of production processes, the reactor, the production efficiency of preparation, and the like. The molding method is also not particularly limited; however, when the carrier, organic auxiliary agent, glass fiber-like inorganic auxiliary agent, binder and the like that will be described below are added to the preliminarily calcined powder and the mixture is molded into a cylindrical shape or an annular shape, a tablet molding machine, an extrusion molding machine or the like is used, and when the mixture is molded into a spherical shape, a granulating machine or the like is used, so as to obtain a molded article. Among these, according to the present invention, a method of adding the glass fiber-like inorganic auxiliary agent together with the preliminarily calcined powder and molding the mixture is preferred; a method of adding the glass fiber-like inorganic auxiliary agent, together with the preliminarily calcined powder, to an inert carrier, and performing supported molding by coating the carrier by a tumbling granulation method, is more preferred; and it is most preferable that an organic auxiliary agent is not added at all when the carrier is coated by a tumbling granulation method.

Regarding the material for the carrier, known materials such as alumina, silica, titania, zirconia, niobia, silica-alumina, silicon carbide, carbides, and mixtures thereof may be used, and there are also no particular limitations on the particle size, water absorption rate, and mechanical strength of the carrier, the degrees of crystallinity or mixing ratio of various crystal phases, and the like. It is particularly preferable that these factors are selected in appropriate ranges in consideration of the performance or moldability of the final catalyst, the production efficiency, and the like. The mixing ratio of the carrier and the preliminarily calcined powder is calculated as the support ratio by the following formula based on the feed weights of the various raw materials.

$$\text{Support ratio (\% by weight)} = (\text{Weight of preliminarily calcined powder used for molding})/\{(\text{weight of preliminarily calcined powder used for molding}) + (\text{weight of carrier used for molding})\} \times 100$$

The amount cf addition of the glass fiber-like inorganic auxiliary agent is preferably from 0.1% by weight to 25% by weight, particularly preferably from 0.3% by weight to 10% by weight, and most preferably from 0.5% by weight to 5% by weight, with respect to the weight of the preliminarily calcined powder. Furthermore, there are also no particular limitations on the material and the component composition of the glass; however, for example, alkali-free glass such as E glass, or glass that has been subjected to various chemical inactivation treatment such as a silane treatment is more preferred from the viewpoint that adverse effects such as the generation of byproducts are not exerted on the catalytic reaction. The glass fiber-like inorganic auxiliary agent may be subjected to a pulverization process before molding, and there are no particular limitations on the method of pulverization; however, pulverization is carried out using, for example, a ball mill, a rod mill, a SAG mill, a jet mill, an autogenous grinding mill, a hammer mill, a pellet mill, a disc mill, a roller mill, a high-pressure grinding mill, and a VSI mill, singly or in combination. The object of this pulverization may be the glass fiber-like inorganic auxiliary agent alone; however, a mixture of the glass fiber-like inorganic auxiliary agent with the catalyst raw materials to be added in the molding step in addition to the preliminarily calcined powder may also be used.

The organic auxiliary agent according to the present invention is an arbitrary powdered, granular, fibrous, or scaly auxiliary agent mainly formed from an organic material that is destroyed by fire by a heat treatment at a temperature of from 200°C to 600°C, the auxiliary agent being partially or entirely destroyed by fire by the main calcination step that will be described below. Examples may include, but are not limited to, polyethylene glycol, polymerization products of various esters, polymer beads, dried bodies of highly water-absorbing resins, water absorbents having arbitrary water absorption rates, various surfactants, various starches such as wheat flour or purified starch, and crystalline or amorphous cellulose and derivatives thereof. It is speculated that if the organic auxiliary agent is destroyed by fire by the main calcination step, and pores are formed in the catalyst as will be described below, on when a load capable of damaging is applied to the catalyst, the stress in the vicinity of the catalyst increases, and consequently, damage is likely to occur. Therefore, it is preferable not to add an organic auxiliary agent at the time of producing the present catalyst. The average particle size of the organic auxiliary agent according to the present invention is in the range of 0.001 to 1,000 with respect to the average particle size of the preliminarily calcined powder.

Here, the binder according to the present invention may be a liquid composed of the compounds that belong to a group of compounds having a molecular diameter in the range of 0.001 or less with respect to the average particle size of the preliminarily calcined powder, singly or in combination. Examples of the binder may include, but are not limited to, the following substances. That is, the binder may be a liquid organic solvent, a dispersion of an organic material, a water-soluble organic solvent, or a mixture of one of those with water at any arbitrary ratio, and there are no particular limitations. However, an aqueous solution of a polyhydric alcohol such as glycerin, or ion-exchanged water is preferred, and ion-exchanged water is most preferred from the viewpoint of moldability. Since the binder includes water or an organic material, the binder is partially or entirely destroyed by fire in the main calcination process that will be described below. Generally, since the molecular diameter of the organic material used for the binder is sufficiently small compared to the average particle size of the preliminarily calcined powder, even if the binder is used, the binder does not induce formation of pores in the catalyst as in the case of the organic auxiliary agent. Even from the findings of the present inventors, although the type of the binder was changed, no significant change in hardness was confirmed. Furthermore, when a solution of the catalyst raw materials described above is used as this binder, it is also possible to introduce elements to the outermost surface of the catalyst according to an embodiment different from Step (A1) .

In regard to the method of supported molding by coating, the amount of use of the binder is preferably from 2 parts by weight to 60 parts by weight, and more preferably from 10 parts by weight to 50 parts by weight, with respect to 100 parts by weight of the preliminarily calcined powder. Since the reaction of the present invention is an exothermic reaction based on oxidative dehydrogenation, supported molding is the most preferred molding method because of the heat dissipation inside the catalyst, and because of the suppression of the production and/or retention of a coke-like material caused by efficiently diffusion of the conjugated diolefin thus produced.

Step (A4) Main calcination

It is preferable that the preliminarily calcined powder or molded article obtained as such is subjected to calcination again (main calcination) at a temperature of from 200°C to 600°C before being used for a reaction. Also in regard to the main calcination, there are no particular limitations on the calcination time or the atmosphere at the time of calcination, and the technique for calcination is also not particularly limited and may be a fluidized bed, a rotary kiln, a muffle furnace, or a tunnel calcination furnace. It is preferable that these are selected in appropriate ranges in consideration of the performance and mechanical strength of the final catalyst, the production efficiency, and the like. Among these, the most preferred according to the present invention is calcination in a tunnel calcination furnace at a temperature in the range of from 300°C to 600°C for from 1 hour to 12 hours in an air atmosphere.

The whole production process according to the present invention means all the processes including from Step (A1) to Step (A4), singly or in combination, starting from the catalyst raw materials to the time point of obtaining the present catalyst. The molding process according to the present invention is a portion or the entirety of Step (A3).

Regarding the catalyst obtained by the preparation described above, there are no particular limitations on the shape or size of the catalyst; however, when the workability of packing into the reaction tube, the pressure loss inside the reaction tube after packing, and the like are taken into consideration, the shape is preferably a spherical shape, the average particle size is preferably from 3.0 mm to 10.0 mm, and the support ratio of the catalytically active component is preferably from 20% by weight to 80% by weight.

The degree of attrition resistance, which is an index representing mechanical strength according to the present invention, is calculated by the following method. Using a tablet abrasion testing machine manufactured by Hayashi Rikagaku Co., Ltd. as the apparatus, 50 g of a catalyst sample is treated at a speed of rotation of 25 rpm for a treatment time of 10 minutes, the fraction that has been abraded is sieved through a standard sieve having a mesh size of 1.70 mm, the weight of the catalyst remaining on the sieve is measured, and the degree of attrition resistance is calculated by the following formula. As the value of the degree of attrition resistance is lower, superior mechanical strength is obtained, and a preferred range of the degree of attrition resistance is, for example, 3% by weight or less, more preferably 1.5% by weight or less, and even mere preferably 0.5% by weight or less.

$$\text{Degree of attrition resistance (\% by weight)} = 100 \times$$

$$[(\text{Weight of catalyst} - \text{weight of catalyst remaining on}$$

$$\text{sieve})/\text{weight of catalyst}]$$

The hardness, which is an index representing the mechanical strength according to the present invention, is calculated by the following method. There are no particular limitations on the apparatus; however, for example, a tensile compression testing machine (TECHNOGRAPH TG5kN) manufactured by Minebea Co., Ltd.) is used, a single catalyst grain is mounted thereon with an attachment for exclusive use connected to the machine, and a displacement-load curve is obtained in a compression mode at a loading rate of 2 mm/min. Mechanical load is applied continuously to the catalyst, and when the load is decreased by 5% or more of the maximum value or is decreased by 0.1 kgf or more, or cracks (fissures) in the catalyst are recognized by visual inspection, the evaluation is instantly stopped. Thus, the maximum value of the displacement-load curve is designated as the hardness of the catalyst. This evaluation is carried out using 30 or more catalyst grains, and the average value thereof is designated as hardness. In the following Examples, regarding the hardness, the hardness obtained by a tensile compression testing machine is described; however, regarding the hardness of the present invention in a broader sense, the mode of measurement does not matter, and any hardness within the range of hardness evaluation by which a significant correlation with the damage of the catalyst caused by a long-term reaction, such as Kiya type hardness or Vickers hardness, will be equally regarded as the hardness. As an index of the mechanical strength, in addition to the average value of hardness of a plurality of catalyst grains as described above, the measure of variations in the hardness of a plurality of catalyst grains, for example, the standard deviation in the case of assuming that the hardness exhibits a normal distribution; the Weibull modulus in the case of assuming that the hardness exhibits the Weibull distribution, the minimum values of hardness of a plurality of catalyst grains, and the like are also applicable.

The conditions for the reaction of producing a conjugated diolefin from a monoolefin having 4 or more carbon atoms by means of the present catalyst are such that as the raw material gas composition, a mixed gas including from 1% by volume to 20% by volume of a monoolefin, from 5% by volume to 20% by volume of molecular oxygen, from 0% by volume to 60% by volume of water vapor, and from 0% by volume to 94% by volume of an inert gas, for example, nitrogen or carbon dioxide, is used, the reaction bath temperature is in the range of from 200°C to 500°C, the reaction pressure is from normal pressure to a pressure of 10 atmospheres, and the space velocity (GHSV) of the raw material gas with respect to the catalyst molded body of the present invention is in the range of from 350 hr$^{-1}$ to 7,000 hr$^{-1}$. Regarding the form of the reaction, there are no limitations among a fixed bed, a mobile bed, and a fluidized bed; however, a fixed bed is preferred.

Damage of a catalyst according to the present invention is a phenomenon in which when the reaction for producing a conjugated diolefin from a monoolefin having 4 or more carbon atoms is carried out as a long-term reaction in the presence of a catalyst, the mechanical strength of the catalyst itself is decreased, the shape is changed or deteriorated from the shape of the catalyst at the time of packing to fragments or even to a powdered form, and the catalyst disintegrates (damaged). Causes for the phenomenon that may be considered include damage from the interior of the catalyst caused by the production of a coke-like material, and/or damage caused by the combustion heat or rapid expansion of the combustion gas caused by a regeneration treatment. As a result of damage of the catalyst, damaged catalyst pieces accumulate inside the reactor, and there is a concern that the accumulation leads to problems such as an increase in the pressure loss, an undesirable reaction caused by the catalyst that has locally accumulated in the reactor, and incorporation of the damaged catalyst pieces into the purification system in the subsequent stages.

The mechanical strength according to the present invention is a collective term for the measurement results obtainable by strength evaluation by means of any physical or mechanical load exerted on a single catalyst grain or a plurality of catalyst grains, such as the degree of attrition resistance and hardness described above, as well as the packing powdering ratio disclosed in Patent Literature 3.

The coke-like material according to the present invention is a material produced by at least any one of the reaction raw materials, the intended product, and reaction byproducts in a reaction for producing a conjugated diolefin. Although the details of the chemical composition of the coke-like material or the production mechanism are not clearly known, the coke-like material is regarded as a causative material that causes various problems, particularly such as inhibition of the flow of a reaction gas in an industrial plant, blocking of the reaction tube, and shoot-down of the reaction resulting therefrom, as the coke-like material is precipitated on or attached to the catalyst surface, an inert material, the interior of the reaction tube, or the interior of the facility of a subsequent process. Furthermore, for the purpose of avoiding the problems described above, generally, in an industrial plant, the reaction is stopped before blocking occurs, and a regeneration treatment of removing the coke-like material by combustion by means of temperature increase at the blocked site in the reaction tube or the facility of a subsequent process, is carried out. Furthermore, regarding the production mechanism for the coke-like material, for example, the following is assumed. That is, at the time of using a composite metal oxide catalyst containing molybdenum, the coke-like material may be produced by a mechanism based on the polymerization of various olefins and the condensation of high-boiling point compounds, both starting from molybdenum compounds that have sublimed and precipitated out in the reactor; a mechanism based on the polymerization of various olefins and the condensation of high-boiling point compounds, both starting from abnormal acid-base points or radical production points in the catalyst and the reactor; or a mechanism based on the production of high-boiling point compounds by the Diels-Alder reaction of conjugated diolefins and other olefin compounds and the condensation at points where the temperature is locally low inside the reactor. In addition to the above-described mechanisms, other various mechanisms are also known.

As will be disclosed below, it is preferable that the present catalyst has a certain degree of attrition resistance and a certain hardness value at least before the initiation of the reaction. As an evaluation method for mechanical strength, which has been hitherto used by those skilled in the art, the degree of attrition resistance that will be described below may be mentioned; however, this is a physical property indicating the extent of damage against impact at the time of catalyst packing, and the problems described below may be considered when the degree of attrition resistance is regarded as a method for evaluating mechanical strength. That is, in the evaluation of the degree of attrition resistance, since the mechanical load exerted to the catalyst is low, there are occasions in which damage is caused by a long-term reaction even in a catalyst having a satisfactory degree of attrition resistance, as will be described below. In this regard, a tensile compression testing machine may exert a mechanical load that is suitably high for the problems of the present invention in connection with the evaluation of hardness, and as will be described below, from the viewpoint that the correlation with damage of the catalyst caused by a long-term reaction may be significantly identified, the tensile compression testing machine is a physical property evaluation method that is more difficult and more suitable than the evaluation of the degree of attrition resistance, which is a conventional evaluation method for mechanical strength. However, as will be described below, it may be seen that even a catalyst having a satisfactory degree of attrition resistance has low hardness and is damaged by a long-term reaction. Thus, it has not been known even to those ordinarily skilled in the art as described above, that hardness evaluation is suitable as a physical property evaluating method indicating the correlation with the damage of catalyst caused by a long-term reaction, as shown by the present invention.

It is known that the degree of attrition resistance is affected by various preparation processes, such as the types and amounts of various strength increasing agents or binders added at the time of molding, or combinations thereof; the atomic ratio of the catalyst composition or the phase morphologies of various crystalline phases, and proportions thereof; the diameter, geometric structure, and aggregate morphology of the secondary particles of the catalytically active components formed in the preparation process or drying process, and the degree of attrition resistance is also in a close relation with the performance of the catalyst. The value of the degree of attrition resistance is preferably 2% by weight or less. Meanwhile, there are no known documents specifically disclosing the findings for increasing hardness particularly in connection with a molded catalyst such as the catalyst of the present invention, and as described above, the present inventors confirmed that hardness increase may be achieved by regulating the type and the amount of addition of an organic auxiliary agent or/and a glass fiber-like inorganic auxiliary agent.

Examples

Hereinafter, the present invention will be described in more detail by way of Examples; however, the present invention is not intended to be limited to the following Examples as long as the gist is maintained. In the following description, unless particularly stated otherwise, percent (%) is used to mean mol%. Furthermore, the definitions of the n-butene conversion ratio, the butadiene yield, and TOS in the following description are as follows.

```
        n-Butene conversion ratio (mol%) = (Number of

    moles of n-butene reacted/number of moles of n-butene

    supplied) × 100


        Butadiene yield (mol%) = (Number of moles of butadiene

    produced/number of moles of n-butene supplied) × 100
```

TOS = Mixed gas flow time (hours)

Example 1

(Preparation of catalyst 1)

800 parts by weight of ammonium heptamolybdate was completely dissolved in 3,000 parts by weight of pure water that was heated to 80°C (stock solution 1) . Next, 11 parts by weight of cesium nitrate was dissolved in 124 ml of pure water, and the solution was added to stock solution 1. Next, 275 parts by weight of ferric nitrate, 769 parts by weight of cobalt nitrate, and 110 parts by weight of nickel nitrate were dissolved in 612 ml of pure water that was heated to 60°C, and the resulting solution was added to the stock solution 1. Subsequently, 165 parts by weight of bismuth nitrate was dissolved in an aqueous solution of nitric acid prepared by adding 42 parts by weight of nitric acid (60% by weight) to 175 ml of pure water that was heated to 60°C, and the resulting solution was added to the stock solution 1. This stock solution 1 was dried by a spray drying method, and the dried powder thus obtained was preliminarily calcined under the conditions of 440°C for 5 hours. To the preliminarily calcined powder obtained in this manner (average particle size: 63.2 $\mu$m, and the atomic ratio calculated from the feed raw materials is Mo : Bi : Fe : Co : Ni : Cs = 12 : 1.7 : 1.8 : 7.0 : 1.0 : 0.15), 5% by weight of crystalline cellulose (average particle size: 89.3 $\mu$m) and 3% by weight of silane-treated glass fibers (average fiber length: 11 $\mu$m, average fiber length: 150 $\mu$m) were added, and the mixture was sufficiently mixed. Subsequently, the mixture was subjected to supported molding by a tumbling granulation method on an inert carrier (silica-alumina) into a spherical shape using a 33 wt% glycerin solution as a binder in an amount of 33% by weight with respect to the preliminarily calcined powder, such that the support ratio would be 50% by weight. The spherically molded article thus obtained was calcined under the conditions of 500°C and 5 hours, and thus catalyst 1 of the present invention was obtained. The degree of attrition resistance of catalyst 1 was 0.20% by weight, and the hardness was 4.5 kgf (44.0 N).

Example 2

(Preparation of catalyst 2)

A catalyst was prepared under the same conditions as the conditions for catalyst 1, except that 5% by weight of crystalline cellulose (average particle size: 89.3 $\mu$m) and 3% by weight of silane-treated glass fibers (average fiber length: 11 $\mu$m, average fiber length: 100 $\mu$m) were added to the preliminarily calcined powder obtained in Example 1, and catalyst 2 of the present invention was obtained. The degree of attrition resistance of catalyst 2 was 0.19% by weight, and the hardness was 3.2 kgf (31.4 N).

Example 3

(Preparation of catalyst 3)

A catalyst was prepared under the same conditions as the conditions for catalyst 1, except that 3% by weight of silane-treated glass fibers (average fiber length: 11 $\mu$m, average fiber length: 150 $\mu$m) were added to the preliminarily calcined powder obtained in Example 1, and thus catalyst 3 of the present invention was obtained. The degree of attrition resistance of catalyst 3 was 0.42% by weight, and the hardness was 15.5 kgf (151.9 N).

Example 4

(Preparation of catalyst 4)

A catalyst was prepared under the same conditions as the conditions for catalyst 1, except that 5% by weight of crystalline cellulose (average particle size: 89.3 $\mu$m) and 3% by weight of glass fibers (average fiber length: 11 $\mu$m, average fiber length: 30 $\mu$m) were added to the preliminarily calcined powder obtained in Example 1, and thus catalyst 4 of the present invention was obtained. The degree of attrition resistance of catalyst 4 was 0.13% by weight, and the hardness was 2.7 kgf (26.5 N).

Example 5

(Preparation of catalyst 5)

A catalyst was prepared under the same conditions as the conditions for catalyst 1, except that 5% by weight of crystalline cellulose (average particle size: 89.3 $\mu$m) and 3% by weight of silane-treated glass fibers (average fiber length: 11 µm, average fiber length: 50 µm) were added to the preliminarily calcined powder obtained in Example 1, and thus catalyst 5 of the present invention was obtained. The degree of attrition resistance of catalyst 5 was 0.56% by weight, and the hardness was 2.9 kgf (28.4 N).

Example 6

(Preparation of catalyst 6)

A catalyst was prepared under the same conditions as the conditions for catalyst 1, except that 5% by weight of crystalline cellulose (average particle size: 89.3 µm) and 3% by weight of silane-treated glass fibers (average fiber length: 11 µm, average fiber length: 3,000 µm) were added to the preliminarily calcined powder obtained in Example 1, and thus catalyst 6 of the present invention was obtained. The degree of attrition resistance of catalyst 6 was 0.15% by weight, and the hardness was 2.5 kgf (24.5 N).

Example 7

(Preparation of catalyst 7)

A catalyst was prepared under the same conditions as the conditions for catalyst 1, except that 5% by weight of crystalline cellulose (average particle size: 89.3 µm) and 3% by weight of silane-treated glass fibers (average fiber length: 10 µm, average fiber length: 300 µm) were added to the preliminarily calcined powder obtained in Example 1, and thus catalyst 7 of the present invention was obtained. The degree of attrition resistance of catalyst 7 was 0.27% by weight, and the hardness was 4.1 kgf (40.2 N) .

Example 8

(Preparation of catalyst 8)

A catalyst was prepared under the same conditions as the conditions for catalyst 1, except that 5% by weight of crystalline cellulose (average particle size: 89.3 µm) and 3% by weight of silane-treated glass fibers (average fiber length: 10 µm, average fiber length: 1,500 µm) were added to the preliminarily calcined powder obtained in Example 1, and thus catalyst 8 of the present invention was obtained. The degree of attrition resistance of catalyst 8 was 0.29% by weight, and the hardness was 2.7 kgf (26.5 N) .

Comparative Example 1

(Preparation of catalyst 9)

A catalyst was prepared under the same conditions as the conditions for catalyst 1, except that 5% by weight of crystalline cellulose (average particle size: 89.3 µm) was added to the preliminarily calcined powder obtained in Example 1, the mixture was sufficiently mixed, and then a 33 wt% glycerin solution was used in an amount of 40% by weight as a binder by a tumbling granulation method, and thus comparative catalyst 9 was obtained. The degree of attrition resistance of catalyst 9 was 0.31% by weight, and the hardness was 1.6 kgf (15.7 N).

Comparative Example 2

(Preparation of catalyst 10)

A catalyst was prepared under the same conditions as the conditions for catalyst 1, except that 5% by weight of crystalline cellulose (average particle size: 89.3 µm) and 3% by weight of talc (average particle size: 57 µm) were added to the preliminarily calcined powder obtained in Example 1, the mixture was sufficiently mixed, and a 33 wt% glycerin solution was used as a binder in the tumbling granulation method in an amount of 40% by weight with respect to the preliminarily calcined powder. Thus, comparative catalyst 10 was obtained. The degree of attrition resistance of catalyst 10 was 0.20% by weight, and the hardness was 1.4 kgf (13.7 N).

Comparative Example 3

(Preparation of catalyst 11)

A catalyst was prepared under the same conditions as the conditions for catalyst 1, except that 3% by weight of talc (average particle size: 57 μm) was added to the preliminarily calcined powder obtained in Example 1, and thus comparative catalyst 11 was obtained. The degree of attrition resistance of catalyst 11 was 0.24% by weight, and the hardness was 1.9 kgf (18.6 N).

Comparative Example 4

(Preparation of catalyst 12)

A catalyst was prepared under the same conditions as the conditions for catalyst 1, except that auxiliary agent was not added to the preliminarily calcined powder obtained in Example 1, and thus comparative catalyst 12 was obtained. The degree of attrition resistance of catalyst 12 was 0.48% by weight, and the hardness was 2.3 kgf (22.5 N).

Test Example 1

(Coke precipitation reaction)

Catalyst 1 obtained in Example 1 was reacted and evaluated by the following method. 53 ml of the catalyst was packed in a stainless steel reaction tube, and a reaction was induced using a mixed gas having a gas volume ratio of 1-butene : oxygen : nitrogen : water vapor = 1 : 1 : 7 : 1 under the conditions of normal pressure and a GHSV of 1,200 hr$^{-1}$, for a TOS of 300 hours by changing the temperature of the reaction bath so that the condition: 1-butene conversion ratio = 80.0 ± 1.0% would be maintained. Thus, a coke-like material was precipitated on the catalyst. A liquid component and a gas component were separated by a condenser at the outlet of the reaction tube, and the various components in the gas component were quantitatively analyzed by a gas chromatograph equipped with a hydrogen flame ionization detector and a gas chromatograph equipped with a thermal conductivity detector. The various data obtained by gas chromatography were processed by factor correction, and the 1-butene conversion ratio and the butadiene selection ratio were calculated. The butadiene selection ratio at a TOS of 280 hours was 88.1%.

(Coke combustion reaction)

After the coke precipitation reaction, for the purpose of combusting the coke-like material precipitated on the catalyst, the coke-like material was combusted by setting the reaction bath temperature at 400°C using a mixed gas having a gas volume ratio of oxygen : nitrogen = 1 : 3 at normal pressure and at a space velocity of 400 hr$^{-1}$ for a TOS of about 10 hours. A quantitative analysis similar to that of the coke precipitation reaction was performed, and it was considered that combustion of the coke-like material was completed at the time point when the amounts of production of $CO_2$ and CO in the reaction tube outlet gas became zero.

(Evaluation of damage ratio)

After the coke combustion reaction, the catalyst obtained after the reaction in the reaction tube was discharged and classified using a sieve having a mesh size of 3.35 mm. Catalyst grains that had been damaged to fragments and powder, which passed through the sieve, were weighed as catalyst pieces, and the damage ratio of the catalyst 1 caused by a long-term test as calculated by the following formula was 0.91% by weight.

```
Damage ratio (% by weight) = Weight of catalyst pieces

(g)/weight of packed catalyst before coke precipitation

reaction (g) × 100
```

Test Example 2

An evaluation of the damage ratio caused by a long-term test was performed under the same reaction conditions as in Test Example 1, except that the catalyst to be evaluated was changed to catalyst 3 obtained in Example 3. The damage ratio of catalyst 3 was 0.12% by weight. The butadiene selection ratio at a TOS of 280 hours was 88.3%.

Comparative Test Example 1

An evaluation of the damage ratio caused by a long-term test was performed under the same reaction conditions as in Test Example 1, except that the catalyst to be evaluated was changed to catalyst 9 obtained in Comparative Example 1. The damage ratio of catalyst 9 was 1.55% by weight. The butadiene selection ratio at a TOS of 280 hours was 87.1%.

Comparative Test Example 2

An evaluation of the damage ratio caused by a long-term test was performed under the same reaction conditions as in Test Example 1, except that the catalyst to be evaluated was changed to catalyst 11 obtained in Comparative Example 3. The damage ratio of catalyst 11 was 1.86% by weight. The butadiene selection ratio at a TOS of 280 hours was 86.6%.

Comparative Test Example 3

An evaluation of the damage ratio caused by a long-term test was performed under the same reaction conditions as in Test Example 1, except that the catalyst to be evaluated was changed to catalyst 12 obtained in Comparative Example 4. The damage ratio of catalyst 12 was 1.16% by weight. The butadiene selection ratio at a TOS of 280 hours was 87.4%.

The results of the degree of attrition resistance, hardness, and damage ratio obtained by Examples, Comparative Examples, Test Examples, and Comparative Test Examples are presented in Table 1. As is obvious from Table 1, the hardness increased as a result of the addition of a glass fiber-like inorganic auxiliary agent according to the present invention, and it is understood that the hardness of a catalyst may be increased more noticeably by not adding an organic auxiliary agent. Furthermore, in a catalyst having increased hardness, the damage ratio caused by a long-term reaction was significantly suppressed, and this suggests that the present catalyst may improve the long-term stability of a reaction. In catalyst 9, damage of the catalyst caused by a long-term reaction was observed, regardless of whether the degree of attrition resistance was satisfactory. Furthermore, from a comparison between catalyst 3 and catalyst 9, since hardness evaluation may significantly correlate hardness with the damage of a catalyst caused by a long-term reaction, it may be concluded that the hardness evaluation is a physical property evaluation method or the long-term stability of a reaction, which is more difficult and more suitable than the evaluation of the degree of attrition resistance. That is, although it has not been known to those ordinarily skilled in the art, it has been found by the present invention that as an evaluation method for a physical property that is correlated with damage caused by a long-term reaction, not the degree of attrition resistance, which is a conventional evaluation method for mechanical strength, but hardness is rather suitable.

[Table 1]

| Catalyst No. | Organic auxiliary agent | Inorganic auxiliary agent | R | Before reaction No. | Before reaction Degree of attrition resistance [wt%] | Before reaction Hardness [kgf] | After reaction No. | After reaction Damage ratio [wt%] |
|---|---|---|---|---|---|---|---|---|
| Catalyst 1 | Crystalline cellulose 5 wt% | Glass fibers (average fiber length:150μm) 3 wt% | 2.37 | Example 1 | 0.20 | 4.5 | Test Example 1 | 0.91 |
| Catalyst 2 | Crystalline cellulose 5 wt% | Glass fibers (average fiber length:100μm) 3 wt% | 1.58 | Example 2 | 0.19 | 3.2 | - | - |
| Catalyst 3 | None | Glass fibers (average fiber length:150μm) 3 wt% | 2.37 | Example 3 | 0.42 | 15.5 | Test Example 2 | 0.12 |
| Catalyst 4 | Crystalline cellulose 5 wt% | Glass fibers (average fiber length:30μm) 3 wt% | 0.47 | Example 4 | 0.13 | 2.7 | - | - |
| Catalyst 5 | Crystalline cellulose 5 wt% | Glass fibers (average fiber length:50μm) 3 wt% | 0.79 | Example 5 | 0.56 | 2.9 | - | - |
| Catalyst 6 | Crystalline cellulose 5 wt% | Glass fibers (average fiber length:3000pm) 3 wt% | 47.47 | Example 6 | 0.15 | 2.5 | - | - |
| Catalyst 7 | Crystalline cellulose 5 wt% | Glass fibers (average fiber length:300μm) 3 wt% | 4.75 | Example 7 | 0.27 | 4.1 | - | - |
| Catalyst 8 | Crystalline cellulose 5 wt% | Glass fibers (average fiber length:1500μm) 3 wt% | 23.73 | Example 8 | 0.29 | 2.7 | - | - |
| Catalyst 9 | Crystalline cellulose 5 wt% | None | - | Comparative Example 1 | 0.31 | 1.6 | Comparative Test Example 1 | 1.55 |
| Catalyst 10 | Crystalline cellulose 5 wt% | Talc (average particle size: 57μm) 3 wt% | 0.90 | Comparative Example 2 | 0.20 | 1.4 | - | - |
| Catalyst 11 | None | Talc (average particle size: 57μm) 3 wt% | 0.90 | Comparative Example 3 | 0.24 | 1.9 | Comparative Test Example 2 | 1.86 |
| Catalyst 12 | None | None | - | Comparative Example 4 | 0.48 | 2.3 | Comparative Test Example 3 | 1.16 |

**Claims**

1.  A molded catalyst for conjugated diolefin production, the catalyst being used for producing a conjugated diolefin by a catalytic oxidative dehydrogenation reaction from a mixed gas including a monoolefin having 4 or more carbon atoms and molecular oxygen, and being obtained by molding a composite metal oxide and a glass fiber-like inorganic auxiliary agent.

2.  The molded catalyst for conjugated diolefin production according to claim 1, wherein the requirement of the following Formula (A) is satisfied:

$$R\ (= La/Dc) \leq 45 \qquad (A)$$

    wherein La represents the average fiber length of the glass fiber-like inorganic auxiliary agent; and Dc represents the average particle size of the composite metal oxide.

3.  The molded catalyst for conjugated diolefin production according to claim 1 or 2, wherein the molded catalyst does not include an organic auxiliary agent.

4.  The molded catalyst for conjugated diolefin production according to any one of claims 1 to 3, wherein the composite metal oxide satisfies the following Compositional Formula (D):

$$Mo_{12}Bi_aFe_bCo_cNi_dX_eY_fZ_g \qquad (D)$$

    wherein X represents at least one element of alkali metals selected from lithium, sodium, potassium, rubidium, and cesium; Y represents at least one element of alkaline earth metals selected from magnesium, calcium, strontium, and barium; Z represents at least one element selected from lanthanum, cerium, praseodymium, neodymium, samarium, europium, antimony, tungsten, lead, zinc, cerium, and thallium; a, b, c, d, e, and f represent the atomic ratios of bismuth, iron, cobalt, nickel, X, Y, and Z, respectively, with respect to molybdenum 12; and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$, g represents a value satisfying the oxidation state of the other elements.

5.  The supported molded catalyst for conjugated diolefin production according to any one of claims 1 to 4, wherein the supported molded catalyst is a catalyst for producing a conjugated diolefin by a catalytic oxidative dehydrogenation reaction from a mixed gas including a monoolefin having 4 or more carbon atoms and molecular oxygen, and is produced by supporting a composite metal oxide and a glass fiber-like inorganic auxiliary agent on a carrier.

6.  A method for producing the molded catalyst for conjugated diolefin production according to any one of claims 1 to 5, the method comprising steps of preparing a mixed solution or slurry including the compounds containing the various metals of the composite metal oxide under the conditions of a temperature of from 20°C to 90°C; subsequently drying and preliminarily calcining the mixture; molding the mixture together with a glass fiber-like inorganic auxiliary agent; and subjecting the molded product to main calcination.

7.  The method for producing the molded catalyst for conjugated diolefin production according to claim 6, wherein the temperature of preliminary calcination is from 200°C to 600°C, and the main calcination temperature is from 200°C to 600°C.

8.  The method for producing the supported molded catalyst for conjugated diolefin production according to claim 6 or 7, the method comprising a molding step of coating a carrier with the composite metal oxide and the glass fiber-like inorganic auxiliary agent together with a binder,
    wherein the support ratio of the catalytically active components is from 20% by weight to 80% by weight, and the average particle size of the molded catalyst is from 3.0 mm to 10.0 mm.

9.  The method for producing the molded catalyst according to any one of claims 6 to 8, wherein an organic auxiliary agent is not used in the entire production process.

**Amended claims under Art. 19.1 PCT**

**1.** A molded catalyst for conjugated diolefin production, the catalyst being used for producing a conjugated diolefin by a catalytic oxidative dehydrogenation reaction from a mixed gas including a monoolefin having 4 or more carbon atoms and molecular oxygen, and being obtained by molding a composite metal oxide and a glass fiber-like inorganic auxiliary agent, wherein the composite metal oxide satisfies the following Compositional Formula (D):

$$Mo_{12}Bi_aFe_bCo_cNi_dX_eY_fZ_g \qquad (D)$$

wherein X represents at least one element of alkali metals selected from lithium, sodium, potassium, rubidium, and cesium; Y represents at least one element of alkaline earth metals selected from magnesium, calcium, strontium, and barium; Z represents at least one element selected from lanthanum, cerium, praseodymium, neodymium, samarium, europium, antimony, tungsten, lead, zinc, cerium, and thallium; a, b, c, d, e, and f represent the atomic ratios of bismuth, iron, cobalt, nickel, X, Y, and Z, respectively, with respect to molybdenum 12; and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$, g represents a value satisfying the oxidation state of the other elements.

**2.** The molded catalyst for conjugated diolefin production according to claim 1, wherein the requirement of the following Formula (A) is satisfied:

$$R \ (= La/Dc) \ \leq \ 45 \qquad (A)$$

wherein La represents the average fiber length of the glass fiber-like inorganic auxiliary agent; and Dc represents the average particle size of the composite metal oxide.

**3.** The molded catalyst for conjugated diolefin production according to claim 1 or 2, wherein the molded catalyst does not include an organic auxiliary agent.

**4.** The supported molded catalyst for conjugated diolefin production according to any one of claims 1 to 3, wherein the supported molded catalyst is a catalyst for producing a conjugated diolefin by a catalytic oxidative dehydrogenation reaction from a mixed gas including a monoolefin having 4 or more carbon atoms and molecular oxygen, and is produced by supporting a composite metal oxide and a glass fiber-like inorganic auxiliary agent on a carrier.

**5.** A method for producing the molded catalyst for conjugated diolefin production according to any one of claims 1 to 4, the method comprising steps of preparing a mixed solution or slurry including the compounds containing the various metals of the composite metal oxide under the conditions of a temperature of from 20°C to 90°C; subsequently drying and preliminarily calcining the mixture; molding the mixture together with a glass fiber-like inorganic auxiliary agent; and subjecting the molded product to main calcination.

**6.** The method for producing the molded catalyst for conjugated diolefin production according to claim 5, wherein the temperature of preliminary calcination is from 200°C to 600°C, and the main calcination temperature is from 200°C to 600°C.

**7.** The method for producing the supported molded catalyst for conjugated diolefin production according to claim 5 or 6, the method comprising a molding step of coating a carrier with the composite metal oxide and the glass fiber-like inorganic auxiliary agent together with a binder,
wherein the support ratio of the catalytically active components is from 20% by weight to 80% by weight, and the average particle size of the catalyst is from 3.0 mm to 10.0 mm.

**8.** The method for producing the molded catalyst according to any one of claims 5 to 7, wherein an organic auxiliary agent is not used in the entire production process.

## INTERNATIONAL SEARCH REPORT

| International application No. |
| --- |
| PCT/JP2016/077314 |

A. CLASSIFICATION OF SUBJECT MATTER
*B01J23/887(2006.01)i, B01J37/02(2006.01)i, B01J37/08(2006.01)i*

According to International Patent Classification (IPC) or to both national classification and IPC

B. FIELDS SEARCHED

Minimum documentation searched (classification system followed by classification symbols)
B01J23/887, B01J37/02, B01J37/08

Documentation searched other than minimum documentation to the extent that such documents are included in the fields searched
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2010-516441 A (BASF SE), | 1-3,5 |
| Y | 20 May 2010 (20.05.2010), | 4,6-9 |
| | claim 20; paragraphs [0042], [0064], [0159], [0169] | |
| | & US 2008/0177105 A1 | |
| | claim 20; paragraphs [0043], [0095], [0227], [0252] | |
| | & WO 2008/087116 A1 & EP 2114562 A1 | |
| Y | WO 2013/161703 A1 (Nippon Kayaku Co., Ltd.), | 4,6-9 |
| A | 31 October 2013 (31.10.2013), | 1-3,5 |
| | claims 1 to 7; paragraphs [0009], [0011] to [0014], [0018], [0024] | |
| | & US 2015/0126774 A1 | |
| | claims 1 to 7; paragraphs [0016], [0021] to [0053], [0060] | |
| | & EP 2842626 A1 | |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 31 October 2016 (31.10.16) | 08 November 2016 (08.11.16) |

| Name and mailing address of the ISA/ | Authorized officer |
| --- | --- |
| Japan Patent Office | |
| 3-4-3,Kasumigaseki,Chiyoda-ku, | |
| Tokyo 100-8915,Japan | Telephone No. |

Form PCT/ISA/210 (second sheet) (January 2015)

**INTERNATIONAL SEARCH REPORT**

International application No.

PCT/JP2016/077314

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2013-202459 A  (Mitsubishi Chemical Corp.), 07 October 2013 (07.10.2013), entire text (Family: none) | 1-9 |
| A | JP 2002-273229 A  (Nippon Shokubai Co., Ltd.), 24 September 2002 (24.09.2002), claims 1 to 4; paragraphs [0014], [0015] & US 2002/0198103 A1 claims 1 to 6; paragraphs [0019], [0020] & EP 1243331 A1 | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011241208 A
- JP 2012046509 A
- JP 3313863 B
- JP 4863436 B
- JP 2002273229 A

- JP 5388897 B
- WO 2012036038 A
- JP 5628936 B
- JP 7016463 A

**Non-patent literature cited in the description**

- **MIYATA, NOBORU ; KANNO, HIROSHI.** *Zairyo (Materials),* vol. 32 (354), 102-108